(19) Europäisches Patentamt
European Patent Office
Office européen des brevets

(11) Veröffentlichungsnummer: **0 000 555**
**B1**

(12) **EUROPÄISCHE PATENTSCHRIFT**

(45) Veröffentlichungstag der Patentschrift: **10.02.82**

(21) Anmeldenummer: **78100457.7**

(22) Anmeldetag: **20.07.78**

(51) Int. Cl.³: **C 07 D 303/08,**
C 07 D 301/14

# (54) Verfahren zur Herstellung von halogenalkylsubstituierten Oxiranen.

(30) Priorität: **28.07.77 DE 2734086**

(43) Veröffentlichungstag der Anmeldung:
**07.02.79 Patentblatt 79/3**

(45) Bekanntmachung des Hinweises auf die Patenterteilung:
**10.02.82 Patentblatt 82/6**

(84) Benannte Vertragsstaaten:
**BE CH DE FR GB NL**

(56) Entgegenhaltungen:
**FR - A - 2 300 085**
**FR - A - 2 309 551**

(73) Patentinhaber: **BAYER AG**
**Zentralbereich Patente, Marken und Lizenzen**
**D-5090 Leverkusen 1, Bayerwerk (DE)**

(73) Patentinhaber: **Degussa Aktiengesellschaft**
**Weissfrauenstrasse 9**
**D-6000 Frankfurt am Main (DE)**

(72) Erfinder: **Rauleder, Gebhard, Dr.**
**Cheruskerstrasse 31**
**D-4000 Düsseldorf (DE)**
Erfinder: **Waldmann, Helmut, Dr.**
**Carl-Rumpff-Strasse 59**
**D-5090 Leverkusen (DE)**
Erfinder: **Hofen, Willi**
**Südring 54**
**D-6451 Rodenbach (DE)**
Erfinder: **Wirthwein, Rolf, Dr.**
**Fürstenberger Strasse 4**
**D-6450 Hanau 9 (DE)**

EP 0 000 555 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von halogenalkylsubstituierten Oxiranen

Die vorliegende Erfindung betrifft ein verbessertes Verfahren zur Herstellung von halogenalkylsubstituierten Oxiranen aus halogenalkylsubstituierten Olefinen und Percarbonsäuren.

Halogenalkylsubstituierte Oxirane finden Verwendung auf dem Gebiet der Lacke und Kunststoffe und als organische Zwischenprodukte.

Es ist bekannt, chloralkylsubstituierte Oxirane aus den entsprechenden Olefinen nach dem Chlorhydrinverfahren herzustellen. Dieses Verfahren hat den Nachteil, daß unerwünschte chlorierte Nebenprodukte und umweltbelastende Salzabfälle gebildet werden (Ullmanns Encyklopädie d. techn. Chemie, Bd. 10, S. 565, linke Spalte, Zeile 1 ff., insbesondere 3. 13—15; DAS 1 543 174, Spalte 2, Zeile 15 ff., insbesondere 3. 32—35).

Häufig ist es auch schwierig, nach der Chlorhydrinmethode definiert ein einziges Reaktionsprodukt herzustellen. So führt die Umsetzung von 4-Chlorbuten-(2) mit unterchloriger Säure zu einem Gemisch von zwei Produkten, die durch nachstehende Formeln gekennzeichnet seien:

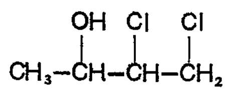

und

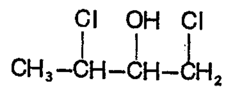

Demgemäß liefert die nachfolgende Dehydrohalogenierung dieses Gemisches mit einer Base ein Gemisch von zwei isomeren Oxiranen, wie nachstehende Formeln veranschaulichen (DAS 1 056 596, Spalte 1, Zeile 53 bis Spalte 2, 3—43):

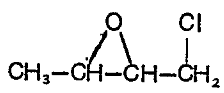

und

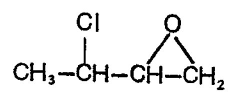

Weiterhin ist es bekannt, Olefine mit Hilfe einer Percarbonsäure in die entsprechenden Oxirane umzuwandeln (N. Prileschajew, Ber. dtsch. Chem. Ges. *42*, 4811 (1909)).

Bei dieser Reaktion handelt es sich um einen elektrophilen Angriff des Oxidationsmittels auf das Olefin. (K. D. Bingham, G. D. Meakins, G. H. Whitham, Chem. Commun. (1966, S 445 und 446).) Aus diesem Grunde nimmt die Reaktivität des Olefins mit fallender Nucleophilie der Doppelbindung ab. Deshalb erschweren elektronegative Substituenten in $\alpha$-Stellung zur C=C—Doppelbindung die Epoxidation. (S. N. Lewis in R. L. Augustin, "Oxidation", Vol. I, Seite 227, insbesondere S. 227, 3. 9—13, Marcel Dekker, New York (1969).) Halogenalkylsubstituierte Olefine lassen sich daher mit Percarbonsäuren nicht ohne weiteres epoxidieren. Infolge der geringen Reaktionsfähigkeit ihrer Doppelbindung sind hohe Temperaturen und lange Reaktionszeiten erforderlich, was zur Bildung von unerwünschten Nebenprodukten wie Dihydroxy- und Hydroxyacyloxy-Derivaten der Ausgangsprodukte Anlaß gibt. (S. N. Lewis in R. L. Augustin, "Oxidation", Vol. I, Seite 233, insbesondere 3. 6—11, Marcel Dekker, New York 1969).

So ist insbesondere im Hinblick auf Art und Durchführung der Reaktion zwischen einem halogenalkylsubstituierten Olefin und einer Percarbonsäure die Struktur und Herstellungsweise der verwendeten Percarbonsäure von großer Bedeutung.

Bekanntlich lassen sich niedere aliphatische Percarbonsäuren in einer Gleichgewichtsreaktion aus Carbonsäure und Wasserstoffperoxid gemäß Gleichung (1) herstellen. (D. Swern, in "Organic Peroxides", Vol. 1, S. 61. Wiley Interscience 1971)

$$RCOOH + H_2O_2 \rightleftharpoons RCOOOH + H_2O \qquad (1)$$

Mit Ausnahme von stärkeren Carbonsäuren wie Ameisensäure und Trifluoressigsäure benötigt man zur schnellen Einstellung des Gleichgewichtes starke Säuren, wie Schwefelsäure, p-Toluolsulfonsäure und andere, als Katalysator (S. N. Lewis in R. L. Augustin "Oxidation", Vol. I, S. 216 ("C. Peracids"), Marcel Dekker, New York 1969). Die Umsetzung von Olefinen mit z. B. nach dieser Methode hergestellter Peressigsäure führte jedoch nicht zu Oxiranen, sondern zu $\alpha$-Glykolen und Hydroxyacetaten (J. Böseken, W. C. Smit und Gaster, Proc. Acad. Sci. Amsterdam, *32* 377—383 (1929).) Die im Reaktionsgemisch vorhandene Mineralsäure katalysiert die Aufspaltung des primär gebildeten Oxirans (D. Swern "Organic Peroxides", Wiley Intersciense 1971. Vol. 2, S. 436), was besonders bei reaktionsträgen Olefinen, wie halogenalkylsubstituierten Olefinen, bei deren Umsetzung hohe Temperaturen und lange Reaktionszeiten nötig sind, zu Oxiran-verlusten führen kann.

Perameisensäure läßt sich ohne zusätzlichen Katalysator aus Wasserstoffperoxid und Ameisensäure herstellen (S. N. Lewis in R. L. Augustin, "Oxidation", Vol. I, S. 217, erster Absatz, Marcel Dekker, New York 1969). Die Umsetzung von $\alpha$-chloralkylsubstituierten Olefinen mit dieser mineralsäurefreien Percarbonsäure lieferte jedoch das entsprechende Epoxid auch nur zu niedrigen Ausbeuten. So wurde zur Epoxidation von 3,4-Dichlorbuten-(1) eine aus 90 %-iger Ameisensaure und 85 %-igem Wasserstoffperoxid hergestellte Perameisensäure verwendet. Nach fünf Stunden Reaktionsdauer bei 60°C wurde 2-(1,2-Dichloräthyl-)oxiran in 30 %-iger Ausbeute erhalten (E. G. E. Hawkins, J. Chem. Soc., 1959 S. 248 bis 256, insbesondere S. 250, Z. 19).

In neuerer Zeit ist ein Verfahren zur Herstellung von aliphatischen Chlorepoxiden durch Umsetzung eines Allylchlorkohlenwasserstoffs, welcher ein Chloratom in Nachbarstellung zur Doppelbindung besitzt, mit einer organischen Perverbindung, die frei von anorganischen Verunreinigungen ist, bekannt geworden (DAS 1 056 596). Die dabei verwendeten Perverbindungen sind "reine Peressigsäure, Perpropionsäure oder Acetaldehydmonoperacetat im Gemisch mit Acetaldehyd und/oder Aceton". (DAS 1 056 596, Spalte 10, Z. 32—35.) Die Epoxidierung gemäß dem Verfahren der DAS 1 056 596 von in Allylstellung chlorsubstituierten Olefinen mit Acetaldehydmonoperacetat liefert die entsprechenden Oxirane in Ausbeuten bezogen auf die Perverbindung je nach Olefin zwischen 17 % und 56 %. (DAS 1 056 596, Spalte 5 bis 7, Zeile 35 ff., Beispiel 1, 3, 4 und 6).

Die bei diesem Verfahren zur Epoxidation verwendete Peressigsäure und Perpropionsäure wird gelöst in einem inerten organischen Lösungsmittel eingesetzt. Wie an anderer Stelle beschrieben, können bei diesem Verfahren als typische inerte Lösungsmittel unter anderem Aceton, Äthylacetat, Butylacetat und Dibutyläther verwendet werden (US—P. 3 150 154, Spalte 3, Zeile 1—3).

Mit den gemäß dem Verfahren der DAS 1 056 596 hergestellten Persäuren lassen sich Allylchlorkohlenwasserstoffe epoxidieren; die Ausbeuten an Oxiranen sind jedoch niedrig; der Persäure-Umsatz ist unvollständig. Er beträgt in den angegebenen Beispielen nur etwa 90 % und die Reinheit der isolierten Oxirane ist für technische Verwendung ungenügend. So wird in der DAS 1 056 596 im Beispiel 5, Spalte 7, Zeile 5 ff. die Epoxidation von 3-Chlor-1-buten mit einer Lösung von Peressigsäure in Aceton beschrieben. Der Persäure-Umsatz beträgt nach zehnstündiger Reaktionsdauer 91 %. Das Oxiran wird mit einer Reinheit von 90,5 % in 68 %-iger Ausbeute isoliert.

In der Brit. PS 784 620 wird im Beispiel VII, Seite 7, Zeile 5 ff., die Herstellung von 3,4-Dichloro-1, 2-epoxybutan durch Umsetzung von 3,4-Dichlor-1-buten mit Peressigsäure in Aceton beschrieben. Danach beträgt der Persäure-Umsatz 89 % und die Epoxid-Ausbeute 75 %. Die Reinheit des Epoxids wird mit 93,3 % angegeben. In der Brit. PS 784 620 wird im Beispiel IX, Seite 7, Zeile 85 ff. auch über die Epoxidation eines Olefines mit Perpropionsäure berichtet. Danach wurde nach der Umsetzung von Crotylchlorid mit einer Lösung von Perpropionsäure in Äthylpropionat das 1-Chlor-2, 3-epoxybutan in 56 %-iger Ausbeute erhalten. Der Persäure-Umsatz betrug 90 %.

Weiterhin werden gemäß der GB—PS 784 620 als Lösungsmittel für Persäuren nur Aceton, Ethylacetat, Essigsäure, Butylacetat und Dibutylether und als Herstellungsmethode für Persäuren nur die Umsetzung von Aldehyden mit Sauerstoff über Peroxide als Zwischenprodukte beschrieben. Benzol ist als Lösungsmittel nicht erwähnt oder nahegelegt, da Benzol im Gegensatz zu den erwähnten Lösungsmitteln unpolar ist. Die Ausbeuten an Dichlorepoxibutanen betragen nach dem Verfahren der GB—PS 75 bzw. 82 % (s. Beispiele III und VII). Diese Ausbeute ist nicht als befriedigend zu bezeichnen und im erfindungsgemäßen Verfahren wesentlich höher.

Schließlich ist aus der DE—OS 26 02 776 (entspricht FR—OS 2 300 085) ein Verfhren zur Epoxidation von Alkenen oder deren Derivaten durch Reaktion mit Persäure bekannt, bei dem man zunächst eine Lösung einer Persäure in einem Chlorkohlenwasserstoff herstellt, diese mit dem Alken oder dessen Derivat im Gleichstrom einem Reaktor zuleitet und die Produktmischung auf spezielle Weise aufarbeitet. Die Reaktions temperatur beträgt bevorzugt etwa 100°C. Wenn längere Reaktionszeiten oder niedrigere Ausbeuten hingenommen werden, kann auch bei 50 bis 150°C gearbeitet werden, anzustreben ist aber ein Bereich von 90 bis 120°C (s. DE—OS 26 02 776, Seiten 10/11). Es ist bekannt, daß Epoxidationen in chlorierten Kohlenwasserstoffen mit wesentlich höherer Reaktionsgeschwindigkeit ablaufen als in Benzol (s. Swern, Organic Peroxides, Volume II, Wiley-Interscience 1971, S. 461 und 464). Es hat deshalb nicht nahegelegen, daß man mit Benzol als Lösungsmittel und bei Temperaturen von 60 bis 80°C halogenalkylsubstituierte Oxirane in Ausbeuten von über 90 % erhalten kann, wie das erfindungsgemäß möglich ist.

Es wurde nun ein Verfahren gefunden zur Herstellung von halogenalkylsubstituierten Oxiranen aus chloralkyloder bromalkylsubstituierten Monoolefinen der allgemeinen Formel

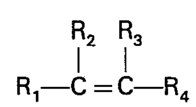

(I)

worin

$R_1$ und $R_4$ unabhängig voneinander Wasserstoff, $C_1$- bis $C_5$-Alkyl, $C_5$- bis $C_7$-Cycloalkyl, Monochlor-$C_1$-

bis $C_5$-Alkyl, Monobrom-$C_1$- bis $C_5$-Alkyl, Dichlor-$C_1$- bis $C_5$-Alkyl, Dibrom-$C_1$- bis $C_5$-Alkyl, Monochlor-$C_5$- bis $C_7$-Cycloalkyl, Monobrom-$C_5$- bis $C_7$-Cycloalkyl, Dichlor-$C_5$- bis $C_7$-Cycloalkyl, Dibrom-$C_5$- bis $C_7$-Cycloalkyl bedeuten,
$R_2$ und $R_3$ unabhängig voneinander für Wasserstoff, $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$-Alkyl und Dibrom-$C_1$- bis $C_5$-Alkyl stehen, wobei die Reste
$R_2$ und $R_3$ zusammen mit den Kohlenstoffatomen der C = C -Doppelbindung einen Ring mit bis zu 12 Kohlenstoffatomen bilden können,
und wobei mindestens einer der Reste $R_1$ bis $R_4$ ein Chlor oder Brom enthaltender Alkyl- oder Cycloalkyl-Rest der genannten Art ist,
und Percarbonsäuren bei erhöhter Temperatur und in Gegenwart von Lösungsmitteln, das dadurch gekennzeichnet ist, daß man die Umsetzung mit einer Lösung einer 3 bis 4 Kohlenstoffatome enthaltenden Percarbonsäure in Benzol bei einem Molverhältnis von Monoolefin zu Percarbonsäure wie 1,1 : 1 bis 10 : 1 und bei einer Temperatur von 60°C bis 80°C umsetzt, wobei die Percarbonsäure bis zu 5 Gew.-% Wasser und bis zu 2 Gew.-% Wasserstoffperoxid enthält und die zur Umsetzung gelangende Percarbonsäurelösung einen Mineralsäuregehalt unterhalb 50 ppm aufweist.

Im Rahmen der Verbindung der Formel (I) kommen beispielsweise Verbindungen der folgenden Formeln besonders in Betracht:

$$R_5\text{—CH=CH—}R_6, \qquad (II)$$

worin
$R_5$ und $R_6$ unabhängig voneinander $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$-Alkyl oder Dibrom-$C_1$- bis $C_5$-Alkyl bedeuten,
wobei die Reste $R_5$ und $R_6$ zusammen mit der Gruppe CH=CH zu einem Ring verknüpft sein können,
und wobei mindestens einer der Reste $R_5$ und $R_6$ Monochlor-$C_1$- bis $C_5$-Alkyl, Monobrom-$C_1$- bis $C_5$-Alkyl, Dichlor-$C_1$- bis $C_5$-Alkyl oder Dibrom-$C_1$- bis $C_5$ Alkyl bedeutet;

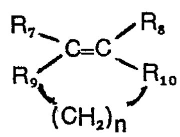
(III)

worin
$R_7$ und $R_8$ unabhängig voneinander Wasserstoff, $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$- Alkyl oder Dibrom-$C_1$- bis $C_5$-Alkyl bedeuten,
$R_9$ und $R_{10}$ unabhängig voneinander einen Methylen-, Chlormethylen-, Brommethylen-, 1,2-Dichloräthylen- oder 1,2-Dibrommethylen-Rest darstellen,
n für eine ganze Zahl von 1 bis 6 steht, und wobei mindestens einer der Reste $R_7$ — $R_{10}$ einen Chlor oder Brom enthaltenden Alkyl-, Cycloalkyl- oder Alkylen-Rest der genannten Art darstellt.

Im einzelnen seien als halogenalkylsubstituierte Monoolefine genannt:

Allylchlorid, 2-Chlormethyl-propen, 3-Chlor-2-chlormethyl-propen, 3-Chlor-1-buten, 1-Chlor-2-buten, 1,4-Dichlor-2-buten, 3,4-Dichlor-1-buten, 3-Chlor-1-penten, 4-Chlor-2-penten, 1-Chlor-2-penten, 1,4-Dichlor-2-penten, 3,4-Dichlor-1-penten, 1,2-Dichlor-3-penten, 3-Chlor-1-cyclopenten, 1,4-Dichlor-2-cyclopenten, 3-Chlor-1-hexen, 1-Chlor-2-hexen, 1,4-Dichlor-2-hexen, 3,4-Dichlor-1-hexen, 2-Chlor-3-hexen, 2,5-Dichlor-3-hexen, 3-Chlor-1-cyclohexen, 1,4-Dichlor-2-cyclohexen, 1-Chlor-2-hepten, 3-Chlor-1-hepten, 3,4-Dichlor-1-hepten, 1,4-Dichlor-2-hepten, 2-Chlor-3-hepten, 2,5-Dichlor-3-hepten, 3-Chlor-1-cyclohepten, 1,4-Dichlor-2-cyclohepten, 1-Chlor-2-octen, 3-Chlor-1-octen, 1,4-Dichlor-2-octen, 2,5-Dichlor-3-octen, 2-Chlor-3-octen, 3-Chlor-4-octen, 3,6-Dichlor-4-octen, 3-Chlor-1-cycloocten, 1,4-Dichlor-2-cycloocten, 1-(1-Chlor-cyclohexyl)-äthen, 1-Chlor-2-nonen, 3-Chlor-1-nonen, 1,4-Dichlor-2-nonen, 2-Chlor-3-nonen, 2,5-Dichlor-3-nonen, 3-Chlor-4-nonen, 6-Chlor-4-nonen, 3,6-Dichlor-4-nonen, 1-Chlor-3-decen, 3-Chlor-1-decen, 4-Chlor-2-decen, 1,4-Dichlor-2-decen, 2-Chlor-3-decen, 2,5-Dichlor-3-decen, 5-Chlor-3-decen, 6-Chlor-4-decen, 3,6-Dichlor-4-decen, 4-Chlor-5-decen, 4,7-Dichlor-5-decen, 1-Chlor-3-undecen, 3-Chlor-1-undecen, 1,4-Dichlor-2-undecen, 2-Chlor-3-undecen, 4-Chlor-2-undecen, 2,5-Dichlor-3-undecen, 5-Chlor-3-undecen, 6-Chlor-4-undecen, 4-Chlor-5-undecen, 4,7-Dichlor-5-undecen, 5-Chlor-6-undecen, 5,8-Dichlor-6-undecen, 1-Chlor-3-dodecen, 3-Chlor-1-dodecen, 1,4-Dichlor-2-dodecen, 2-Chlor-3-dodecen, 4-Chlor-2-dodecen, 2,5-Dichlor-3-dodecen, 5-Chlor-3-dodecen, 6-Chlor-4-dodecen, 4-Chlor-5-dodecen, 4,7-Dichlor-5-dodecen, 5-Chlor-6-dodecen, 5,8-Dichlor-6-dodecen, 5,7-Dichlor-6-dodecen, 7-Chlor-5-dodecen; Allylbromid, 2-Brommethyl-propen, 3-Brom-2-brommethyl-propen, 3-Brom-1-buten, 1-Brom-2-buten, 1,4-Dibrom-2-buten, 3,4-Dibrom-1-buten, 3-Brom-1-penten, 4-Brom-2-penten, 1-Brom-2-penten, 1,4-Dibrom-2-penten, 3,4-Dibrom-1-penten, 1,2-Dibrom-3-penten, 3-Brom-1-cyclopenten, 1,4-Dibrom-2-cyclopenten, 3-Brom-1-hexen, 1-Brom-2-hexen, 1,4-Dibrom-2-hexen, 3,4-Dibrom-1-hexen,

2-Brom-3-hexen, 2,5-Dibrom-3-hexen, 3-Brom-1-cyclohexen, 1,4-Dibrom-2-cyclohexen, 1-Brom-2-hepten, 3-Brom-1-hepten, 3,4-Dibrom-1-hepten, 1,4-Dibrom-2-hepten, 2-Brom-3-hepten, 2,5-Dibrom-3-hepten, 3-Brom-1-cyclohepten, 1,4-Dibrom-2-cyclohepten, 1-Brom-2-octen, 3-Brom-1-octen, 1,4-Dibrom-2-octen, 2,5-Dibrom-3-octen, 2-Brom-3-octen, 3-Brom-4-octen, 3,6-Dibrom-4-octen, 3-Brom-1-cycloocten, 1,4-Dibrom-2-cycloocten, 1-(1-Brom-cyclohexyl)-äthen, 1-Brom-2-nonen, 3-Brom-1-nonen, 1,4-Dibrom-2-nonen, 2-Brom-3-nonen, 2,5-Dibrom-3-nonen, 3-Brom-4-nonen, 6-Brom-4-nonen, 3,6-Dibrom-4-nonen, 1-Brom-3-decen, 3-Brom-1-decen, 4-Brom-2-decen, 1,4-Dibrom-2-decen, 2-Brom-3-decen, 2,5-Dibrom-3-decen, 5-Brom-3-decen, 6-Brom-4-decen, 3,6-Dibrom-4-decen, 4-Brom-5-decen, 4,7-Dibrom-5-decen, 1-Brom-3-undecen, 3-Brom-1-undecen, 1,4-Dibrom-2-undecen, 2-Brom-3-undecen, 4-Brom-2-undecen, 2,5-Dibrom-3-undecen, 5-Brom-3-undecen, 6-Brom-4-undecen, 4-Brom-5-undecen, 4,7-Dibrom-5-undecen, 5-Brom-6-undecen, 5,8-Dibrom-6-undecen, 1-Brom-3-dodecen, 3-Brom-1-dodecen, 1,4-Dibrom-2-dodecen, 2-Brom-3-dodecen, 4-Brom-2-dodecen, 2,5-Dibrom-3-dodecen, 5-Brom-3-dodecen, 6-Brom-4-dodecen, 4-Brom-5-dodecen, 4,7-Dibrom-5-dodecen, 5-Brom-6-dodecen, 5,8-Dibrom-6-dodecen, 5,7-Dibrom-6-dodecen, 7-Brom-5-dodecen.

Besonders geeignet zur Umsetzung mit Percarbonsäuren gemäß dem erfindungsgemäßen Verfahren sind chloralkyl- oder brom-alkylsubstituierte Monoolefine der Formel

$$R_{11}—CH=CH—R_{12}, \qquad (IV)$$

worin

$R_{11}$ und $R_{12}$ unabhängig voneinander Wasserstoff, $C_1$- bis $C_5$-Alkyl, Monochlor-$C_1$- bis $C_5$-Alkyl, Monobrom-$C_1$-bis $C_5$-Alkyl, Dichlor-$C_1$- bis $C_5$-Alkyl oder Dibrom-$C_1$- bis $C_5$-Alkyl bedeuten,

wobei mindestens einer der Reste $R_{11}$ und $R_{12}$ für Monochlor-$C_1$- bis $C_5$-Alkyl, Monobrom-$C_1$- bis $C_5$-Alkyl, Dichlor-$C_1$- bis $C_5$-Alkyl oder Dibrom-$C_1$- bis $C_5$-Alkyl steht.

Im einzelnen seien beispielsweise genannt: Allylchlorid, 2-Chlormethyl-propen, 3-Chlor-2-chlormethyl-propen, 3-Chlor-1-buten, 1-Chlor-2-buten, 1,4-Dichlor-2-buten, 3,4-Dichlor-1-buten, 3-Chlor-1-penten, 4-Chlor-2-penten, 1-Chlor-2-penten, 1,4-Dichlor-2-penten, 3,4-Dichlor-1-penten, 1,2-Dichlor-3-penten, 3-Chlor-1-hexen, 1-Chlor-2-hexen, 1,4-Dichlor-2-hexen, 3,4-Dichlor-1-hexen, 2-Chlor-3-hexen, 2,5-Dichlor-3-hexen, 3-Chlor-1-cyclohexen, 1,4-Dichlor-2-cyclohexen; Allylbromid, 2-Brommethyl-propen, 3-Brom-2-brommethyl-propen, 3-Brom-1-buten, 1-Brom-2-buten, 1,4-Dibrom-2-buten, 3,4-Dibrom-1-buten, 3-Brom-1-penten, 4-Brom-2-penten, 1,4-Dibrom-2-penten, 3,4-Dibrom-1-penten, 1,2-Dibrom-3-penten, 3-Brom-1-hexen, 1-Brom-2-hexen, 1,4-Dibrom-2-hexen, 3,4-Dibro-1-hexen, 2-Brom-3-hexen, 2,5-Dibrom-3-hexen, 3-Brom-1-cyclohexen, 1,4-Dibrom-2-cyclohexen.

Ganz besonders sind zur Umsetzung mit Percarbonsäuren nach dem erfindungsgemäßen Verfahren 1,4-Dichlor-2-buten, 1,4-Dibrom-2-buten und 3,4-Dichlor-1-buten geeignet.

Als Lösungsmittel wird im erfindungsgemäßen Verfahren Benzol eingesetzt.

Erfindungsgemäß verwendbare Persäuren sind Perpropionsäure, Perbuttersäure und Perisobuttersäure. Bevorzugt verwendet werden Perpropionsäure und Perisobuttersäure. Besonders bevorzugt ist Perpropionsäure. Die Herstellung der mineralsäurefreien Persäuren in Benzol kann z.B. nach dem in der DOS 2 262 970 beschriebenen Verfahren erfolgen.

Das erfindungsgemäße Verfahren wird bei 60—80°C, besonders bevorzugt bei 65—75°C durchgeführt. In Sonderfällen können die angegebenen Temperaturen unter- oder überschritten werden.

Neben der Arbeitsweise unter isothermen Bedingungen, d. h. Einhaltung einer einheitlichen Temperatur im gesamten Reaktionsgemisch, kann man die Umsetzung auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das Molverhältnis von Olefin zu Persäure beträgt erfindungsgemäß 1,1 : 1 bis 10 : 1. Bevorzugt wird ein Molverhältnis von 1,25 : 1 bis 5 : 1 angewendet. Ganz besonders vorteilhaft ist es, ein Molverhältnis von 1,5 bis 3,0 Mol Olefin je Mol Persäure anzuwenden.

Das erfindungsgemäße Verfahren kann bei den verschiedensten Drücken durchgeführt werden. Im allgemeinen arbeitet man bei Normaldruck; das Verfahren kann jedoch auch bei Unter- oder Überdruck durchgeführt werden.

Der Wassergehalt der zur Epoxidation verwendeten Percarbonsäure beträgt bis zu 5 Gew.-%. Geeignet ist beispielsweise eine Percarbonsäure mit einem Wassergehalt von bis zu 2 Gew.-%. Vorzugsweise verwendet man eine Percarbonsäurelösung, die weniger als 1 Gew.-% Wasser enthält. Besonders bevorzugt ist ein Wassergehalt von weniger als 0,1 Gew.-%.

Der Wasserstoffperoxidgehalt der angewendeten Percarbonsäure beträgt bis zu 2 Gew.-%. Vorteilhaft arbeitet man bei einem Gehalt von weniger als 1 Gew.-%. Besonders vorteilhaft ist es, die Umsetzung mit einer Percarbonsäurelösung durchzuführen, die einen Wasserstoffperoxidgehalt unterhalb 0,3 % aufweist.

Das erfindungsgemäße Verfahren wird mit einer Percarbonsäurelösung durchgeführt, die einen Mineralsäuregehalt unterhalb 50 ppm besitzt. Besonders vorteilhaft ist ein Mineralsäuregehalt von weniger als 10 ppm.

Die Durchführung der Reaktion kann diskontinuierlich oder koninuierlich in den für Umsetzungen dieser Art üblichen Vorrichtungen wie Rührwerkskessel, Sidereaktoren, Röhrenreaktoren, Schlaufenreaktoren oder Schleifenreaktoren erfolgen.

Als Werkstoffe für die Durchführung der Verfahren können Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die die Schwermetallionen durch Komplexbildung inaktivieren. Bekannte Substanzen solcher Art sind Gluconsäure, Äthylendiamintetraessigsäure, Natriumsilicat, Natriumpyrophosphat, Natriumhexametaphosphat, Dinatriumdimethylpyrophosphat oder $Na_2$(2-Äthyl-hexyl)$_5$($P_3O_{10}$)$_2$ (DAS 1 056 596, Spalte 4, Zeile 60 ff.).

Das halogenalkylsubstituierte Olefin kann auf vershiedene Art in die für die Umsetzung verwendete Vorrichtung eingebracht werden. Man kann es gemeinsam mit der Percarbonsäurelösung in den Reaktor geben oder man führt die beiden Komponenten getrennt voneinander dem Reaktor zu. Weiterhin ist es möglich, das Olefin und die Percarbonsäurelösung an verschiedenen Stellen in die Reaktoreinheit zu leiten. Bei Verwendung mehrerer in Kaskade geschalteter Reaktoren kann es zweckmäßig sein, das gesamte Olefin in den ersten Reaktor einzubringen. Man kann aber das Olefin auch auf die verschiedenen Reaktoren aufteilen.

Die Reaktionswärme wird durch innen- oder außenliegende Kühler abgeführt. Zur Ableitung der Reaktionswärme kann die Umsetzung auch unter Rückfluß (Siedereaktoren) durchgeführt werden.

Die Reaktion wird zweckmäßigerweise unter möglichst vollständiger Umsetzung der Percarbonsäure vorgenommen. Im allgemeinen setzt man mehr als 95 Mol-% der Percarbonsäure um. Zweckmäßig ist es, mehr als 98 Mol-% Persäure umzusetzen.

Bei der erfindungsgemäßen Durchführung der Reaktion zwischen dem halogenalkylsubstituierten Olefin und der Persäure gelingt es, Oxiranausbeuten von 90 % der Theorie und darüber, bezogen auf eingesetzte Percarbonsäure, zu erzielen.

Die Aufarbeitung des Reaktionsgemisches erfolgt in an sich bekannter Weise, z. B. durch Destillation. Besonders vorteilhaft ist es, das Reaktionsgemisch vor der destillativen Aufarbeitung zur Abtrennung der bei der Umsetzung gebildeten, der Percarbonsäure entsprechenden Carbonsäure mit Wasser zu extrahieren. Die Durchführung der Extraktion kann in den üblichen Extraktoren, wie Mischer-Scheider, Siebbodenextraktoren, pulsierende Siebbodenkolonnen, Drehscheibenextraktoren oder Extraktionszentrifugen erfolgen.

Bei einer bevorzugten Durchführung des Verfahrens wird eine etwa 20 Gew.-%ige Perpropionsäurelösung in Benzol unter Rühren zu der dreifach-molaren Menge halogenalkylsubstituiertem Olefin, das auf 70°C thermostatisiert ist, gegeben. Die Perpropionsäurelösung enthält weniger als 10 ppm Mineralsäure; sie hat einen Wassergehalt, der unter-halb von 0,1 % liegt und weist einen Wasserstoffperoxidgehalt von weniger als 0,3 % auf. Zur Komplexierung von Schwermetallionen wurde der Perpropionsäure vor der Umsetzung etwa 0,05 Gew.-% $Na_5$(2-Äthylhexyl)$_5$($P_3O_{10}$)$_2$ zugesetzt. Der Fortgang und das Ende der Reaktion werden kontrolliert, indem man der Reaktionslösung in zeitlichen Abständen Proben entnimmt und titrimetrisch den noch vorhandenen Gehalt an Percarbonsäure bestimmt. Nach Beendigung der Reaktion wird das Reaktionsgemisch abgekühlt und zur Entfernung der Propionsäure dreimal mit derselber Gewichtsmenge Wasser gewaschen. Anschließend wird das propionsäurefreie Reaktionsgemisch fraktioniert.

Mit dem erfindungsgemäßen Verfahren können halogenalkylsubstituierte Oxirane in hohen Ausbeuten und großer Reinheit hergestellt werden.

Die folgenden Beispiele erläutern die Erfindung. Sämtliche Prozentangaben stellen, soweit nichts anderes gesagt wird, Gewichtsprozent dar.

Beispiel 1.

Herstellung von Epichlorhydrin aus Allylchlorid und Perpropionsäure.

In einem 200 ml Dreihals-Doppelwandkolben mit Magnetrührer, Innenthermometer, Tropftrichter und Rückflußkühler wurden 45 g 20 %ige Perpropionsäure in Benzol (0,1 Mol) vorgelegt und auf 70°C thermostatisiert. Danach tropft man 22,96 g 10,3 Mol) Allylchlorid so zu, daß die Temperatur bei 70°C gehalten werden konnte. Nach weiteren 6 Stunden Rühren unter Rückfluß ergab die titrimetrische Analyse einen Perpropionsäure-Umsatz von 98,5 %. Die gaschromatographische Analyse des Reaktionsgemisches zeigte, daß Epichlorhydrin mit einer Selektivität von 97,5 %, bezogen auf eingesetzte Perpropionsäure, gebildet wurde. Nach dem Abkühlen wurde das Reaktionsgemisch zur Entfernung der Propionsäure mehrmals mit Wasser gewaschen und anschließend fraktioniert. Es resultierten 8,73 g Epichlorhydrin.

Beispiel 2.

Herstellung von 2-(1,2-Dichloräthyl-)oxiran aus 3,4-Dichlor-1-buten und Perpropionsäure.

In 55,87 g (0,447 Mol) 3,4-Dichlor-1-buten tropfte man bei 70°C unter Rühren 62,96 g (0,147 Mol) Perpropionsäure als 21 %ige Lösung in Benzol. Nach Zutropfen wurde noch weitere 6 Stunden bei dieser Temperatur gerührt, dann zeigte die titrimetrische Analyse einen Perpropionsäure-Umsatz von 97 %. Die Reaktionslösung wurde auf Raumtemperatur abgekühlt und gaschromatographisch analysiert. Wie die Analyse zeigte, wurde 2-(1,2-Dichloräthyl-)-oxiran mit einer Selektivität, bezogen auf eingesetzte Perpropionsäure, von 94,2 % gebildet.

Das Reaktionsgemisch wurde zur Entfernung der Propionsäure mehrmals mit Wasser gewaschen, Benzol abdestilliert und anschließend in einer 10 cm Füllkörper-Kolonne, gefüllt mit 4 mm Glas-Raschingringen, fraktioniert. Es wurden 18,9 g 2-(1,2-Dichloräthyl-)-oxiran mit einer Reinheit von 99,4 % isoliert.

Beispiel 3.

Herstellung von 2,3-Bis-(chlormethyl)-oxiran aus 1,4-Dichlor-2-buten und Perpropionsäure.

Zu 55,2 g (0,4416 Mol) 1,4-Dichlor-2-buten wurden bei 70°C 63,97 g (0,147 Mol) Perpropionsäure als 20,68 %ige Lösung in Benzol zugetropft und anschließend bei dieser Temperatur weitergerührt. Nach 4 Stunden Reaktionszeit betrug der Persäure-Umsatz 96 %, nach 6 Stunden über 99 %. 2,3-Bis(chlormethyl)-oxiran wurde mit einer Selektivität, bezogen auf eingesetzte Perpropionsäure, von 96,5 % gebildet. Nach Entfernen der Propionsäure durch mehrmaliges Ausschüttein des Reaktionsgemisches mit Wasser und anschließendes Abtrennen von Benzol durch Destillation wurden nach der Fraktionierung über eine 30 cm Füllkörperkolonne, gefüllt mit 4 mm Glasraschigringen, 19,6 g 2,3-Bis-(chlormethyl)-oxiran mit einer Reinheit von 99,9 % erhalten.

Beispiel 4.

Kontinuierliche Herstellung von 2,3-Bis-(chlormethyl-oxiran aus 1,4-Dichlor-2-buten und Perpropionsäure.

Eine Lösung von Perpropionsäure in Benzol, die mit einem Stabilisator vom Typ der im Handel erhältlichen Natriumsalze von mit langkettigen Alkoholen partiell veresterten Polyphosphorsäuren versetzt ist, wurde mit 1,4-Dichlor-2-buten in einem als dreistufige Rührkesselkaskade ausgebildeten Reaktionssystem umgesetzt. Jeder der drei Rührkessel hatte ein Volumen von 10 l. Die Beheizung der Kessel erfolgte über im Kessel angebrachte Heizschlangen. Alle drei Kessel wurden auf 70°C thermostatisiert.

Diesem Reaktionssystem wurden pro Stunde 2.137,5 g Perpropionsäure als 20 %ige Lösung in Benzol (4,75 Mol) und 1.781,25 g (14,25 Mol) 1,4-Dichlor-2-buten zugeführt, was einer mittleren Verweilzeit von etwa 8 Stunden entsprach. Unter diesen Reaktionsbedingungen wurde die Perpropionsäure zu 96,8 % umgesetzt. Die Selektivität des gebildeten 2,3-Bis-(Chlormethyl)-oxirans betrug 96 %, bezogen auf eingesetzte Perpropionsäure.

Das hinter dem dritten Reaktor anfallende reaktionsgemisch besaß im Mittel folgende Zusammensetzung: 35,6 % Benzol, 30,8 % 1,4-Dichlor-2-buten, 16,24 % 2,3-Bis-(chlormethyl)-oxiran und 17 % Propionsäure. Dieses Gemisch wurde zur Abtrennung der Propionsäure in einer pulsierenden Siebbodenkolonne mit der zweifachen Menge Wasser extrahiert. Danach betrug der Restgehalt an Propionsäure 0,04 %. Das nach dieser Operation erhaltene Reaktionsgemisch wurde in einer Destillationsstraße aufgetrennt. In einer ersten Kolonne wurde Benzol in einer Menge von 1.395 g pro Stunde destilliert. Das Sumpfprodukt dieser Kolonne, das im wesentlichen aus Ausgangsprodukt und Oxiran bestand, wurde in einer zweiten Kolonne unter vermindertem Druck aufgetrennt. Als Kopfprodukt wurden pro Stunde 1.206,5 g 1,4-Dichlor-2-buten erhalten. Das Sumpfprodukt dieser Kolonne wurde in einer dritten Kolonne unter vermindertem Druck von Hochsiedern befreit. Als Kopfprodukt wurden pro Stunde 629,5 g 2,3-Bis-(chlormethyl)-oxiran mit einer Reinheit von über 99,9 % erhalten. Dem entspricht eine Ausbeute von 94 %, bezogen auf die in das Reaktionssystem eingesetzte Perpropionsäure.

Beispiel 5.

Herstellung von 2,3-Bis-(brommethyl)-oxiran aus 1,4-Dibrom-2-buten und Perpropionsäure.

a) Bromierung von Butadien

171,4 g (3,17 Mol) Butadien wurden in 400 ml n-Hexan gelöst. Bei —20°C wurden unter Rühren 314 g (1,964 Mol) Brom zugetropft. Nach Zutropfende wurden noch 2 Stunden bei dieser Temperatur nachgerührt. Anschließend ließ man auf Raumtemperatur erwärmen und entfernte das Lösungsmittel im Vakuum. Es resultierten 380,4 g rohes Dibrombuten, wobei das Verhältnis 1,4-Dibrom-2-buten zu 3,4-Dibrom-1-buten etwa 2:1 war. Die Isolierung des 1,4-Dibrom-2-butens erfolgte durch Destillation.

b) Umsetzung von 1,4-Dibrom-2-buten mit Perpropionsäure

Zu 64,2 g (0,3 Mol) 1,4-Dibrom-2-buten wurde bei 70°C unter Rühren 45 g 20 %ige Perpropionsäure in Benzol (0,1 Mol) getropft und noch weitere 4 Stunden bei dieser Temperatur gerührt. Nach dieser Zeit betrug der Persäure-Umsatz 95 %. Die gaschromatographische Analyse ergab, daß das Epoxid mit einer Selektivität von 92,6%, bezogen auf eingesetzte Perpropionsäure, gebildet wurden. Das Reaktionsgemisch wurde nach dem Abkühlen zur Entfernung der Propionsäure mehrmals mit Wasser gewaschen und fraktioniert. Es wurden 20.7 g Epoxic erhalten.

**Patentansprüche**

1. Verfahren zur Herstellung von halogenalkylsubstituierten Oxiranen aus chlor-alkyl- oder bromalkylsubstituierten Monoolefinen der allgemeinen Formel

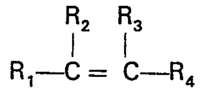

worin
.$R_1$ und $R_4$ unabhängig voneinander Wasserstoff, $C_1$- bis $C_5$-Alkyl, $C_5$ bis $C_7$-Cycloalkyl, Monochlor-$C_1$- bis $C_5$-Alkyl, Monobrom-$C_1$- bis $C_5$-Alkyl, Dichlor-$C_1$- bis $C_5$-Alkyl, Dibrom-$C_1$- bis $C_5$-Alkyl, Monochlor-$C_5$- bis $C_7$-Cycloalkyl, Monobrom-$C_5$- bis $C_7$-Cycloalkyl, Dichlor-$C_5$- bis $C_7$-Cyclo-alkyl, Dibrom-$C_5$- bis $C_7$-Cycloalkyl bedeuten,
$R_2$ und $R_3$ unabhängig voneinander für Wasserstoff, $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$-Alkyl und Dibrom-$C_1$- bis $C_5$-Alkyl stehen, wobei die Reste
$R_2$ und $R_3$ zusammen mit den Kohlenstoffatomen der C = C -Doppelbindung einen Ring mit bis zu 12 Kohlenstoffatomen bilden können,
und wobei mindestens einer der Reste $R_1$ bis $R_4$ ein Chlor oder Brom enthaltender Alkyl- oder Cycloalkyl-Rest der genannten Art ist.
und Percarbonsäuren bei erhöhter Temperatur und in Gegenwart von Lösungsmitteln, dadurch gekennzeichnet, daß man die Umsetzung mit einer Lösung einer 3 bis 4 Kohlenstoffatome enthaltenden Percarbonsäure in Benzol bei einem Molverhältnis von Monoolefin zu Percarbonsäure von 1,1 : 1 bis 10 : 1 und bei einer Temperatur von 60 bis 80°C durchführt, wobei die Percarbonsäure bis zu 5 Gew.-% Wasser und bis zu 2 Gew.-% Wasserstoffperoxid enthält und die zur Umsetzung gelangende Percarbonsäurelösung einen Mineralsäuregehalt unterhalb von 50 ppm besitzt.

2. Verfahren nach Anspruch 1, dadurch gekennzeichnet, daß man als chloralkyl- oder bromalkylsubstituiertes Monoolefin ein Olefin der Formel

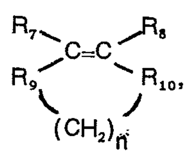

worin
$R_7$ und $R_8$ unabhängig voneinander Wasserstoff, $C_1$- bis $C_5$-Alkyl, Monochlor-$C_1$- bis $C_5$-Alkyl, Monobrom-$C_1$- bis $C_5$-Alkyl, Dichlor-$C_1$- bis $C_5$-Alkyl oder Dibrom-$C_1$ bis $C_5$-Alkyl bedeuten,
$R_9$ und $R_{10}$ unabhängig voneinander einen Methylen-, Chlormethylen-, Brommethylen-, 1,2-Dichloräthylen oder 1,2-Dibrommethylen-Rest darstellen,
n für eine ganze Zahl von 1 bis 6 steht, und wobei mindestens einer der Reste $R_7$ — $R_{10}$ einen Chlor oder Brom enthaltenden Alkyl-, Cycloalkyl- oder Alkylen-Rest der genannten Art darstellt,
einsetzt.

3. Verfahren nach Anspruch 1, dadurch gekennzeichnet, daß man als chloralkyl- oder bromalkylsubstituiertes Olefin 1,4-Dichlor-2-buten einsetzt.

4. Verfahren nach Anspruch 1, dadurch gekennzeichnet, daß man als chloralkyl- oder bromalkylsubstituiertes Olefin 3,4-Dichlor-1-buten einsetzt.

5. Verfahren nach Anspruch 1, dadurch gekennzeichnet, daß man als chloralkyl- oder bromalkylsubstituiertes Olefin 1,4-Dibrom-2-buten einsetzt.

6. Verfahren nach Anspruch 1 bis 5, dadurch gekennzeichnet, daß man als Percarbonsäure Perpropionsäure einsetzt.

7. Verfahren nach Anspruch 1 bis 5, dadurch gekennzeichnet, daß man als Percarbonsäure Perisobuttersäure einsetzt.

8. Verfahren nach Anspruch 1 bis 7, dadurch gekennzeichnet, daß man die Umsetzung bei einem Molverhältnis von Olefin zur Persäure wie 1,5 bis 3 : 1 durchführt.

9. Verfahren nach Anspruch 1 bis 8, dadurch gekennzeichnet, daß man das Reaktionsgemisch vor der destillativen Aufarbeitung zur Abtrennung der bei der Umsetzung gebildeten, der Percarbonsäure entsprechenden Carbonsäure, mit Wasser extrahiert.

## Claims

1. Process for the preparation of halogenalkyl-substituted oxiranes from chloroalkyl-substituted or bromoalkyl-substituted monoolefins of the general formula

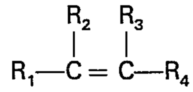

wherein

$R_1$ and $R_4$ independently of one another denote hydrogen, $C_1$- to $C_5$-alkyl, $C_5$- to $C_7$-cycloalkyl, monochloro-$C_1$- to $C_5$-alkyl, monobromo-$C_1$- to $C_5$-alkyl, dichloro-$C_1$- to $C_5$-alkyl, dibromo-$C_1$- to $C_5$-alkyl, monochloro-$C_5$- to $C_7$-cycloalkyl, monobromo-$C_5$- to $C_7$-cycloalkyl, dichloro-$C_5$- to $C_7$-cycloalkyl or dibromo-$C_5$- to $C_7$-cycloalkyl and

$R_2$ and $R_3$ independently of one another represent hydrogen, $C_1$- to $C_5$-alkyl, monochloro-$C_1$- to $C_5$-alkyl, monobromo-$C_1$- to $C_5$-alkyl, dichloro-$C_1$- to $C_5$-alkyl and dibromo-$C_1$- to $C_5$-alkyl, it being possible for the radicals

$R_2$ and $R_3$, together with the carbon atoms of the C=C double bond, to form a ring with up to 12 carbon atoms, and at least one of the radicals $R_1$ to $R_4$ being an alkyl or cycloalkyl radical of the type mentioned containing chlorine or bromine,

and percarboxylic acids at an elevated temperature and in the presence of solvents, characterised in that the reaction is carried out with a solution of a percarboxylic acid containing 3 to 4 carbon atoms in benzene, at a molar ratio of monoolefin to percarboxylic acid of 1.1 : 1 to 10 : 1 and at a temperature of 60°C to 80°C, the percarboxylic acid containing up to 5 % by weight of water and up to 2 % by weight of hydrogen peroxide and the percarboxylic acid solution used for the reaction having a mineral acid content of below 50 ppm.

2. Process according to Claim 1, characterised in that an olefin of the formula

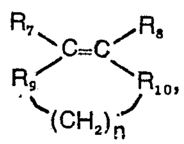

wherein

$R_7$ and $R_8$ independently of one another denote hydrogen, $C_1$- to $C_5$-alkyl, monochloro-$C_1$- to $C_5$-alkyl, monobromo-$C_1$- to $C_5$-alkyl, dichloro-$C_1$- to $C_5$-alkyl or dibromo-$C_1$- to $C_5$-alkyl,

$R_9$ and $R_{10}$ independently of one another represent a methylene, chloromethylene, bromomethylene, 1,2-dichloroethylene, or 1,2-dibromomethylene radical and

n represents an integer from 1 to 6, at least one of the radicals $R_7$—$R_{10}$ representing an alkyl, cycloalkyl or alkylene radical of the type mentioned containing chlorine or bromine,

is employed as the chloroalkyl-substituted or bromoalkyl-substituted monoolefin.

3. Process according to Claim 1, characterised in that 1,4-dichloro-2-butene is employed as the chloroalkyl-substituted or bromoalkyl-substituted olefin.

4. Process according to Claim 1, characterised in that 3,4-dichloro-1-butene is employed as the chloroalkyl-substituted or bromoalkyl-substituted olefin.

5. Process according to Claim 1, characterised in that 1,4-dibromo-2-butene is employed as the chloroalkyl-substituted or bromoalkyl-substituted olefin.

6. Process according to Claim 1 to 5, characterised in that perpropionic acid is employed as the percarboxylic acid.

7. Process according to Claim 1 to 5, characterised in that perisobutyric acid is employed as the percarboxylic acid.

8. Process according to Claim 1 to 7, characterised in that the reaction is carried out at a molar ratio of olefin to peracid of 1.5 to 3 : 1.

9. Process according to Claim 1 to 8, characterised in that the reaction mixture is extracted with water, before working up by distillation, in order to separate off the carboxylic acid, corresponding to the percarboxylic acid, which is formed during the reaction.

**Revendications**

1. Procédé de production d'oxiranes à substituants halogénalkyle à partir de mono-oléfines à substituants chloralkyle ou bromalkyle de formule générale:

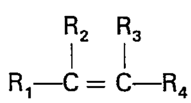

dans laquelle:

$R_1$ et $R_4$ désignent, indépendamment l'un de l'autre, de l'hydrogène, un reste alkyl en $C_1$ à $C_5$, cyclo-

alkyle en $C_5$ à $C_7$, monochloralkyle en $C_1$ à $C_5$, monobromalkyle en $C_1$ à $C_5$, dichloralkyle en $C_1$ à $C_5$, dibromalkyle en $C_1$ à $C_5$, monochlorocycloalkyle en $C_5$ à $C_7$, monobromocyclo-alkyle en $C_5$ à $C_7$, dichlorocyclo-alkyle en $C_5$ à $C_7$, dibromocyclo-alkyle en $C_5$ à $C_7$,

$R_2$ et $R_3$ désignent, indépendamment l'un de l'autre, l'hydrogène, un reste alkyle en $C_1$ à $C_5$, monochloralkyle en $C_1$ à $C_5$, monobromalkyle en $C_1$ à $C_5$, dichloralkyle en $C_1$ à $C_5$ et dibromalkyle en $C_1$ à $C_5$, les restes

$R_2$ et $R_3$ pouvant former conjointement avec les atomes de carbone de la double liaison C=C un noyau ayant jusqu'à 12 atomes de carbone, et au moins l'un des restes $R_1$ à $R_4$ étant un reste alkyl ou cycloalkyle du type mentionné contenant du chlore ou du brome,

et d'acides percarboxyliques à température élevée et en présence de solvants, caractérisé en ce qu'on conduit la réaction avec une solution d'un acide percarboxylique contenant 3 ou 4 atomes de carbone dans le benzène dans un rapport molaire de la mono-oléfine à l'acide percarboxylique de 1,1 : 1 à 10 : 1 et à une température de 60 à 80°C, l'acide percarboxylique contenant jusqu'à 5 % en poids d'eau et jusqu'à 2 % en poids de peroxyde d'hydrogène et la solution d'acide percarboxylique amenée à réagir ayant une teneur en acide minéral inférieure à 50 ppm.

2. Procédé suivant la revendication 1, caractérisé en ce qu'on utilise comme mono-oléfine à substituant chloralkyle ou bromalkyle une oléfine de formule:

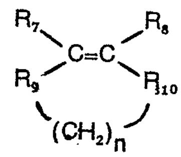

dans laquelle:

$R_7$ et $R_8$ désignent, indépendamment l'un de l'autre, de l'hydrogène, un reste alkyle en $C_1$ à $C_5$, monochloralkyle en $C_1$ à $C_5$, monobromalkyle en $C_1$ à $C_5$, dichloralkyle en $C_1$ à $C_5$ ou dibromalkyle en $C_1$ à $C_5$,

$R_9$ et $R_{10}$ désignent, indépendamment l'un de l'autre, un reste méthylène, chlorométhylène, bromométhylène, 1,2-dichloréthylène ou 1,2-dibromométhylène,

*n* désigne un nombre entier de 1 à 6 et au moins l'un des restes $R_7$—$R_{10}$ représente un reste alkyle, cycloalkyle ou alkylène du type mentionné contenant du chlore ou du brome.

3. Procédé suivant la revendication 1, caractérisé en ce qu'on utilise le 1,4-dichloro-2-butène comme oléfine à substituant chloralkyle ou bromalkyle.

4. Procédé suivant la revendication 1, caractérisé en ce qu'on utilise le 3,4-dichloro-1-butène comme oléfine à substituant chloralkyle ou bromalkyle.

5. Procédé suivant la revendication 1, caractérisé en ce qu'on utilise le 1,4-dibromo-2-butène comme oléfine à substituant chloralkyle ou bromalkyle.

6. Procédé suivant les revendications 1 à 5, caractérisé en ce qu'on utilise comme acide percarboxylique l'acide perpropionique.

7. Procédé suivant les revendications 1 à 5, caractérisé en ce qu'on utilise comme acide percarboxylique l'acide perisobutyrique.

8. Procédé suivant les revendications 1 à 7, caractérisé en ce qu'on conduit la réaction à un rapport molaire de l'oléfine au peracide de 1,5 à 3 : 1.

9. Procédé suivant les revendications 1 à 8, caractérisé en ce qu'on extrait le mélange réactionnel avec de l'eau avant le traitement par distillation pour la séparation de l'acide carboxylique formé lors de la réaction, correspondant à l'acide percarboxylique.